(19) Europäisches Patentamt
European Patent Office
Office européen des brevets

(11) Veröffentlichungsnummer: **0 113 043**
**B1**

(12) # EUROPÄISCHE PATENTSCHRIFT

(45) Veröffentlichungstag der Patentschrift: **11.09.85**

(51) Int. Cl.⁴: **C 07 C 119/048**, C 08 G 18/76, C 08 G 18/75

(21) Anmeldenummer: **83111852.6**

(22) Anmeldetag: **26.11.83**

(54) **Diisocyanate oder Diisocyanat-Gemische, Verfahren zu ihrer Herstellung, sowie ihre Verwendung zur Herstellung von Polyurethankunststoffen.**

(30) Priorität: **08.12.82 DE 3245320**

(43) Veröffentlichungstag der Anmeldung: **11.07.84 Patentblatt 84/28**

(45) Bekanntmachung des Hinweises auf die Patenterteilung: **11.09.85 Patentblatt 85/37**

(84) Benannte Vertragsstaaten: **BE DE FR GB IT**

(56) Entgegenhaltungen: **US - A - 3 663 514**

(73) Patentinhaber: **BAYER AG, Konzernverwaltung RP Patentabteilung, D-5090 Leverkusen 1 Bayerwerk (DE)**

(72) Erfinder: **Knöfel, Hartmut, Dr., Dülmener Weg 21, D-5068 Odenthal-Erberich (DE)**
Erfinder: **Brockelt, Michael, Neuenhauser Weg 1, D-5060 Bergisch Gladbach (DE)**
Erfinder: **Penninger, Stefan, Dr., Pommernallee 5, D-4047 Dormagen 1 (DE)**
Erfinder: **Stutz, Herbert, Dr., Mörikestrasse 22, D-4047 Dormagen 5 (DE)**

**EP 0 113 043 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue, am aromatischen Ring mono- bzw. di-substituierte, gegebenenfalls als Isomerengemische vorliegende Isocyanatobenzyl-cyclohexylisocyanate, ein Verfahren zu ihrer Herstellung durch Phosgenierung der ihnen zugrundeliegenden Diamine bzw. Diamingemische, sowie ihre Verwendung als Aufbaukomponente bei der Herstellung von Polyurethankunststoffen.

Asymmetrische Diisocyanate, welche eine aromatisch und eine cycloaliphatisch gebundene Isocyanatgruppe aufweisen, eignen sich wegen der stark unterschiedlichen Reaktivität dieser Isocyanatgruppen ausgezeichnet zur Herstellung von Polyurethankunststoffen nach dem Prepolymerverfahren, wobei zunächst die höherreaktive, aromatisch gebundene Isocyanatgruppe unter Bildung des NCO-Prepolymeren abreagiert, welches dann in einer zweiten Reaktionsstufe zum hochmolekularen Polyurethan umgesetzt wird, oder zur Herstellung von modifizierten Diisocyanaten, beispielsweise von Uretdion-diisocyanaten, wobei auch hier in einer ersten Reaktionsstufe die höherreaktive Isocyanatgruppe unter Dimerisierung abreagiert, so daß ein Uretdiongruppen aufweisendes Diisocyanat erhalten wird, welches mit Verbindungen gegenüber Isocyanatgruppen reaktionsfähigen Gruppen weiter umgesetzt werden kann. Die US-PS 3 663 514 beschreibt nun bereits ein derartiges Diisocyanat, nämlich das 4-(4-Isocyanato-benzyl)-cyclohexylisocyanat, jedoch scheiterte bislang ein großtechnischer Einsatz dieses Diisocyanats an dem Umstand, daß das, dem Diisocyanat zugrundeliegende, Diamin durch asymetrische Kernhydrierung nur in Ausbeuten von weniger als 35% der Theorie zugänglich und daher das reine asymetrische Diisocyanat nur mit erheblichem Reinigungsaufwand zu gewinnen ist.

Es war die der vorliegenden Erfindung zugrundeliegende Aufgabe, neue Diisocyanate mit aromatisch und cycloaliphatisch gebundenen Isocyanatgruppen aufzufinden, die möglichst bei Raumtemperatur flüssig und niedrigviskos sein sollten, die bezüglich Löslichkeit und Verträglichkeit mit den, Hydroxylgruppen aufweisenden, Reaktionspartnern den Forderungen der technischen Polyurethanchemie genügen, die nur geringe Verunreinigungen an unhydrierten bzw. perhydrierten Diisocyanaten aufweisen, und deren Herstellung mit hohen Selektivitäten, d. h. in hohen Ausbeuten, gelingt.

Diese Aufgabe konnte überraschenderweise durch die Bereitstellung der nachstehend näher beschriebenen Diisocyanate bzw. Diisocyanatgemische und des zu ihrer Herstellung geeigneten Verfahrens gelöst werden.

Gegenstand der Erfindung sind gegebenenfalls Isomerengemische darstellende und gegebenenfalls im Gemisch mit untergeordneten Mengen an den entsprechenden perhydrierten Diisocyanaten und/oder im Gemisch mit untergeordneten Mengen an den entsprechenden unhydrierten aromatischen Diisocyanaten vorliegende Diisocyanate der Formel

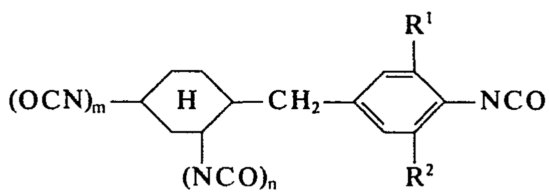

in welcher

$R^1$ und $R^2$ jeweils gleich oder verschieden sind und Wasserstoff oder (gegebenenfalls verzweigte) Alkylgruppen mit 1 bis 12 Kohlenstoffatomen bedeuten mit der Maßgabe, daß mindestens einer der Reste $R^1$ und $R^2$ für einen Alkylrest steht und

m und n jeweils für 0 oder 1 stehen, mit der Maßgabe, daß die Summe $m+n$ den Wert 1 ergibt, wobei im Falle von m oder $n=0$ die verbleibende freie Valenz durch Wasserstoff abgesättigt ist.

Gegenstand der Erfindung ist auch ein Verfahren zur Herstellung dieser Diisocyanate bzw. Diisocyanatgemische, dadurch gekennzeichnet, daß man, die ihnen zugrundeliegenden, gegebenenfalls als Stellungs- und/oder Stereoisomerengemisch und gegebenenfalls im Gemisch mit untergeordneten Mengen an den entsprechenden perhydrierten Diaminen und/oder gegebenenfalls im Gemisch mit untergeordneten Mengen an den entsprechenden unhydrierten aromatischen Diaminen vorliegenden Aminobenzyl-cyclohexylamine in an sich bekannter Weise bei $-20$ bis $+250^\circ$ C phosgeniert.

Gegenstand der Erfindung ist auch die Verwendung der erfindungsgemäßen Diisocyanate als Ausgangsmaterialien bei der Herstellung von Polyurethankunststoffen nach dem Isocyanat-Polyadditionsverfahren.

Ausgangsmaterialien für das erfindungsgemäße Verfahren zur Herstellung der neuen Diisocyanate sind, gegebenenfalls als Stellungs- und/oder Stereoisomerengemische vorliegende Diamine der allgemeinen Formel

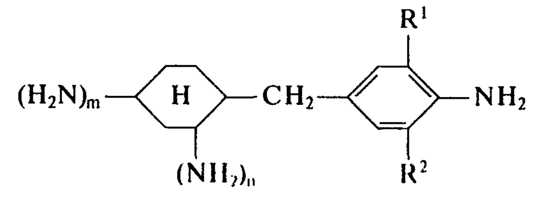

in welcher

$R^1$, $R^2$, m und n die bereits o. g. Bedeutung haben, wobei jedoch die Alkylgruppen vorzugsweise 1 bis 4 Kohlenstoffatome aufweisen und besonders bevorzugt Methylgruppen darstellen.

Die beim erfindungsgemäßen Verfahren einzusetzenden Diamine bzw. Diamingemische stellen oftmals Gemische mit untergeordneten Mengen an den entsprechenden perhydrierten und/oder mit untergeordneten Mengen an den entsprechenden unhydrierten aromatischen Diaminen dar. Unter »untergeordneten Mengen« ist hierbei im allgemeinen ein Anteil dieser perhydrierten bzw. unhydrierten Diamine von jeweils max. 15, vorzugsweise max. 5 Gew.-%, bezogen auf Gesamtgemisch, zu verstehen. Die Hauptkomponente bzw. die Hauptkomponenten der beim erfindungsgemäßen Verfahren einzusetzenden Diamine bzw. Diamingemische, d. h. die Aminobenzyl-cyclohexylamine stellen nach NMR-spektroskopischem Befund praktisch ausschließlich asymmetrische Diamine der angegebenen Struktur dar, in welchem die Alkylsubstituenten mit dem aromatischen Ring verknüpft sind. Die Zusammensetzung der erfindungsgemäßen Diisocyanate bzw. der erfindungsgemäßen Diisocyanatgemische entspricht der genannten Zusammensetzung der beim erfindungsgemäßen Verfahren einzusetzenden Diamine bzw. Diamingemische.

Die Herstellung der beim erfindungsgemäßen Verfahren einzusetzenden Diamine erfolgt vorzugsweise durch partielle Kernhydrierung der ihnen zugrundeliegenden alkylsubstituierten Diaminodiphyenylmethane. Diese asymmetrisch substituierten Diaminodiphenylmethane können beispielsweise durch Kondensation von o- und/oder p-Nitrobenzylhalogeniden, insbesondere Chloriden, mit substituierten Anilinen der Formel

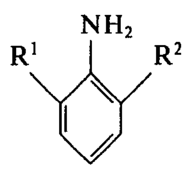

Reduktion der Nitrogruppe zum primären Amin und anschließende Umlagerung in Analogie zu BE-PS 864 533 oder GB-PS 1 567 114 erhalten werden. Je nach dem, ob bei der Kondensationsreaktion reines o-Nitrobenzylhalogenid bzw. reines p-Nitrobenzylhalogenid bzw. Gemische dieser Isomeren eingesetzt werden, entstehen letztlich reine Isomere oder Isomerengemische darstellende Diamine der o. g. allgemeinen Formel, wobei das Verhältnis der 4,4'-Diamino-3(,5)-(di)-alkyldiphenylmethane zu den 4,2'-Diamino-3(,5)-(di)-alkyldiphenylmethane weitgehend dem bei der Kondensationsreaktion eingesetzten Isomerenverhältnis p-Nitrobenzylhalogenid : o-Nitrobenzylhalogenid entspricht. Lediglich bei Verwendung von monosubstituierten Anilinen ($R^2 = H$) entstehen untergeordnete Mengen (bis zu 5 Gew.-%, bezogen auf Gesamtgemisch) an 2,4'-Diamino-3-alkyldiphenylmethanen und/oder an den entsprechenden 2,2'-Isomeren.

Die Kernhydrierung der aromatischen Diamine erfolgt nach den bekannten Methoden des Standes der Technik (vgl. US-PS 2 511 028). Hierbei werden die aromatischen Diamine unter Anlagerung von 3 Mol Wasserstoff pro Mol Diamin katalytisch hydriert. Dies bedeutet, daß die Hydrierungsreaktion vorzugsweise nach Verbrauch von 3 Mol Wasserstoff pro Mol Ausgangsverbindung abgebrochen wird.

Die Hydrierung erfolgt bei 20 bis 300° C, vorzugsweise bei 70 bis 300° C und insbesondere bei 120 bis 250° C unter einem Druck von 20 bis 300 bar, vorzugsweise 70 bis 300 bar, insbesondere 120 bis 250 bar.

Die Hydrierungsreaktion erfolgt in Gegenwart von 0,1 bis 10 Gew.-%, vorzugsweise von 0,1 bis 1 Gew.-%, bezogen auf katalytisch wirksames Metall einerseits und Diaminoverbindungen andererseits, eines Hydrierungskatalysators. Geeignete Katalysatoren sind beispielsweise, gegebenenfalls auf inerten Trägern wie Aktivkohle, Kieselgel und insbesondere Aluminiumoxid, vorliegende Elemente der 8. Nebengruppe des Periodensystems der Elemente oder katalytisch wirksame organische Verbindungen dieser Elemente. Besonders gut geeignet sind z. B. Ruthenium-, Platin-, Rhodium-, Nickel- und/oder Kobaltkatalysatoren in elementarer oder chemisch gebundener Form. Besonders bevorzugt werden Ruthenium oder katalytisch wirksame Rutheniumverbindungen eingesetzt. Beispiele geeigneter Rutheniumverbindungen sind Rutheniumoxid, Rutheniumtetroxid, Bariumperruthenit, Natrium-, Kalium-, Silber-, Calcium- oder Magnesiumruthenat, Natriumperruthenat, Rutheninumpentafluorid, Rutheniumtetrafluoridhydrat oder Rutheniumtrichlorid. Falls Trägersubstanzen für die Katalysatoren

mitverwendet werden, beträgt der Metallgehalt des Trägerkatalysators im allgemeinen 1 bis 10 Gew.-%, vorzugsweise 1 bis 5 Gew.-%, im übrigen ist selbstverständlich die Art und Menge des einzusetzenden Katalysators keineswegs erfindungswesentlich, da die Hydrierungsreaktionen nach den an sich bekannten Methoden des Standes der Technik erfolgt.

Es ist oft zweckmäßig, die Hydrierungsreaktion in Gegenwart von Ammoniak durchzuführen, da Ammoniak unerwünschte Desaminierungsreaktionen und die Bildung von sekundären Aminen als Nebenprodukte unterdrückt. Falls Ammoniak mitverwendet wird, geschieht dies in Mengen von 0,1 bis 30 Gew.-%, vorzugsweise 5 bis 10 Gew.-%, bezogen auf zu hydrierende Ausgangsmaterialien.

Die Hydrierung kann lösungsmittelfrei oder in Gegenwart inerter Lösungsmittel durchgeführt werden. Im allgemeinen werden niedrigschmelzende bzw. flüssige Diamine in Substanz, hochschmelzende Diamine dagegen in gelöster Form hydriert. Als Lösungsmittel eignen sich unter den Reaktionsbedingungen inerte organische Verbindungen mit niedrigem Siedepunkt, vorzugsweise Alkohole wie Methanol, Ethanol, n-Propanol, i-Propanol oder Ether wie z. B. Dioxan, Tetrahydrofuran oder Diethylether oder Kohlenwasserstoffe wie Cyclohexan. Die Durchführung der Hydrierung findet z. B. kontinuierlich in einem Reaktionsrohr, einer Druckkesselkaskade oder vorzugsweise diskontinuierlich in einem Rührautoklaven statt, indem man den Autoklaven mit Katalysator, der zu hydrierenden Substanz und gegebenenfalls einem Lösungsmittel beschickt, mehrmals mit Inertgas spült und gegebenenfalls Ammoniak zudosiert. Danach drückt man Wasserstoff auf, bringt das Gemisch auf Reaktionstemperatur und hydriert bis die theoretisch erforderliche Wasserstoffmenge absorbiert ist. Nach Abkühlung des Reaktionsgemischs und Abtrennung des Katalysators kann das Hydrierungsprodukt destillativ aufgearbeitet werden.

Die Hydrierungsprodukte fallen mit hohen Ausbeuten an, die bei monoalkylsubstituierten Diaminen über 70% der Theorie und bei dialkylsubstituierten Diaminen über 90% der Theorie betragen können und lassen sich durch Destillation nahezu vollständig von nicht umgesetzten aromatischen Diaminen oder von den als Nebenprodukt entstehenden perhydrierten Diaminen abtrennen. Sie stellen im allgemeinen Stereo-, gegebenenfalls auch Stellungsisomerengemische dar und entsprechen bezüglich der Stellungsisometrie weitgehend den Ausgangsmaterialien. Eine Auftrennung in einzelne Stellungs- und/oder Stereoisomere ist im allgemeinen bezüglich der technischen Verwendbarkeit der Hydrierungsprodukte als Ausgangsmaterialien für das erfindungsgemäße Verfahren nicht erforderlich, da es hierbei nicht auf Isomerenreinheit ankommt und im Gegenteil häufig Isomerengemische erwünscht sind, da diese die Eigenschaften der Diisocyanate verbessern.

Bevorzugte, beim erfindungsgemäßen Verfahren einzusetzende, gemäß dem beschriebenen Hydrierungsverfahren zugängliche Ausgangsmaterialien sind beispielsweise 4-(4-Amino-3,5-dimethylbenzyl)-cyclohexylamin, 4-(4-Amino-3,5-diethylbenzyl)-cyclohexylamin, 4-(4-Amino-3,5-diisopropylbenzyl)-cyclohexylamin, 4-(4-Amino-3-ethyl-5-methylbenzyl)-cyclohexylamin, sowie diese Diamine als wesentliche Komponente enthaltende Isomerengemische; 2-(4-Amino-3,5-dimethylbenzyl)-cyclohexylamin, 2-(4-Amino-3,5-diethylbenzyl)-cyclohexylamin, 2-(4-Amino-3,5-diisopropylbenzyl)-cyclohexylamin, 2-(4-Amino-3-ethyl-5-methylbenzyl)-cyclohexylamin sowie diese Diamine als wesentliche Komponente enthaltende Isomerengemische; 4-(4-Amino-3-methylbenzyl)-cyclohexylamin, 4-(4-Amino-3-ethylbenzyl)-cyclohexylamin; 4-(4-Amino-3-isopropylbenzol)-cyclohexylamin und deren Gemischen mit anderen Stellungsisomeren, wie z. B. den entsprechenden 2-(4-Amino-3-alkylbenzyl)-cyclohexylaminen und/oder 4-(2-Amino-3-alkylbenzylcyclohexylaminen; 2-(4-Amino-3-methylbenzyl)-cyclohexylamin, 2-(4-Amino-3-ethylbenzyl)-cyclohexylamin, 2-(4-Amino-3-isopropylbenzyl)-cyclohexylamin sowie deren Gemische mit den entsprechenden 4-(4-Amino-3-alkylbenzyl)- bzw. 4-(2-Amino-3-alkylbenzyl)-cyclohexylaminen.

Bei der Durchführung des erfindungsgemäßen Verfahrens zur Herstellung der neuen Diisocyanate erfolgt die Phosgenierung der beispielhaft genannten Diamine oder deren Salze nach an sich bekannten Verfahren in Gegenwart eines inerten organischen Lösungsmittels (vgl. Houben-Weyl, Methoden der organischen Chemie, Georg Thieme Verlag Stuttgart (1952), Band 8, 4. Auflage, Seiten 120 ff).

Als zu phosgenierende Salze eignen sich vorzugsweise die Hydrochloride oder Ammoniumcarbonate, die durch Sättigung der Diaminlösungen mit gasförmigem Chlorwasserstoff bzw. Kohlendioxid anfallen. Prinzipiell können auch andere Salze, die beispielsweise durch Neutralisation der Diamine mit Protonen abspaltenden Säuren anfallen, phosgeniert werden.

Die Selektivität der Phosgenierungsreaktion ist weitgehend von der Aminokonzentration und vom Phosgenüberschuß abhängig. Vorzugsweise wird das Phosgen in einem hohen molaren Überschuß und das zu phosgenierende Diamin in stark verdünnter Form eingesetzt. Im allgemeinen beträgt der molare Phosgenüberschuß 100 bis 2000%, vorzugsweise 100 bis 1000% und die Aminokonzentration, bezogen auf die Gesamtmenge an Amino einerseits und Lösungsmittel andererseits, bei 0,1 bis 15 Gew.-%, vorzugsweise 5 bis 10 Gew.-%.

Als Lösungsmittel können beliebige inerte organische Flüssigkeiten oder deren Gemische mit einem Siedepunkt von 60 bis 250°C, d. h. Halogenkohlenwasserstoffe, Aromaten, Hydroaromaten sowie deren Chlorverbindungen verwendet werden. Als Beispiele geeigneter Lösungsmittel seien Xylol, Mesitylen, Chlorbenzol, Dichlorbenzol, Trichlorbenzol, Chlornaphtalin und Dichlorethan genannt.

Die Reaktion wird entweder einstufig durch Heißphosgenierung bei Temperaturen von 100 bis 250°C oder zweistufig durch Kalt/Heißphosgenierung bei Temperaturen von —20 bis 250°C unter Normaldruck durchgeführt.

Bei Verwendung der freien Amine als Ausgangsverbindung (Basenphosgenierung) wird zuerst bei Temperaturen von —20 bis +60°C Ammoniumcarbaminsäurechlorid hergestellt, das dann bei Temperaturen von 20 bis 250°C mit Phosgen zum Diisocyanat weiterreagiert.

Die Reinigung der Verfahrensprodukte erfolgt im allgemeinen nach Entphosgenierung durch Abdampfen des Lösungsmittels und anschließende Destillation unter vermindertem Druck.

Die erfindungsgemäßen Verfahrensprodukte, d. h. die neuen erfindungsgemäßen Diisocyanate fallen in hohen Ausbeuten als farblose, niedrigviskose Flüssigkeiten an und stellen wertvolle Aufbaukomponenten bei der Herstellung von Polyurethankunststoffen nach dem Isocyanat-Polyadditionsverfahren dar. Die Stellungs- und/oder Stereoisomerie der neuen Diisocyanate entspricht der Isomerie der bei der Phosgenierung eingesetzten Diamine. Im allgemeinen ist es nicht erforderlich, die beim erfindungsgemäßen Verfahren anfallenden Gemische in einzelne Stellungs- und/oder Stereoisomere aufzutrennen, da die erfindungsgemäßen Verfahrensprodukte ohne derartige Trennungsorperation direkt der erfindungsgemäßen Verwendung zugeführt werden können. Die neuen erfindungsgemäßen Diisocyanate können besonders vorteilhaft zur Herstellung von Polyurethanlacken, Polyurethanelastomeren oder Polyurethanschaumstoffen eingesetzt werden, wobei sie bei den an sich bekannten Verfahren zur Herstellung derartiger Kunststoffe anstelle oder zusammen mit den bislang eingesetzten Polyisocyanaten zum Einsatz gelangen. Besonders vorteilhaft werden die neuen erfindungsgemäßen Diisocyanate bzw. Diisocyanatgemische bei der Herstellung von Polyurethankunststoffen der beispielhaft genannten Art nach dem Prepolymerprinzip verwendet.

Die nachfolgenden Beispiele dienen der weiteren Erläuterung der Erfindung. Alle Prozentangaben beziehen sich, soweit nichts anderslautendes vermerkt, auf Gewichtsprozente. Die Analyse der Isomerenverteilung der Zwischen- und Endprodukte erfolgte gaschromatographisch.

### Beispiel 1

a) 250 g (1,04 Mol) 4,4'-Diamino-3-ethyl-5-methyldiphenylmethan und 25 g Ruthenium-Aluminumoxid-Trägerkatalysator (5% Ru auf $Al_2O_3$) werden in einem 0,7 l Rührautoklaven vorgelegt und nach mehrmaligem Spülen mit Stickstoff und Wasserstoff mit 25 g Ammoniak versetzt. Man erhitzt unter Rühren auf 150 bis 155°C und hydriert bei 200 bar bis 3,12 Mol Wasserstoff aufgenommen worden sind. Danach läßt man abkühlen, entspannt den Autoklaven und löst das Rohprodukt in Methanol auf. Der Katalysator wird abfiltriert, mit Methanol gewaschen und die organischen Lösungen vereinigt. Nach dem Abdampfen des Lösungsmittels wird das Produkt einer Flash-Destillation unterworfen und anschließend fraktioniert destilliert. Dabei werden 220 g Diamin gewonnen, das einen Siedepunkt von 125 bis 130°C/0,05 mbar aufweist und laut gaschromatographischem Befund zu 97,2% aus 4-(4-Amino-3-ehtyl-5-methylbenzyl)-cyclohexylamin, 0,8% aus 4,4'-Diamino-3-ethyl-5-methyldicyclohexylmethan und zu 2,0% aus nicht umgesetztem Ausgangsprodukt und unbekanntem Diamin besteht.

b) Man löst bei —5 bis 8°C 250 g Phosgen in 700 ml Chlorbenzol und läßt unter Rühren eine Lösung von 123 g des in Beispiel 1a) beschriebenen Diamins in 700 ml Chlorbenzol zutropfen. Dabei entsteht eine Suspension, die sich auf 20°C erwärmt. Man erhitzt unter Einleiten von 100 g/h Phosgen auf 130°C, wobei der Feststoff in Lösung geht und kocht weitere 2 Stunden am Rückfluß. Danach wird die Phosgenzufuhr beendet, überschüssiges Phosgen mit Stickstoff ausgeblasen und das Rohprodukt durch Destillation gereinigt. Dabei fallen 139 g (96% der Theorie) 4-(4-Isocyanato-3-ethyl-5-methylbenzyl)-cyclohexylisocyanat an. Das Produkt ist durch folgende Daten charakterisiert:

| | |
|---|---|
| Siedepunkt: | 133 bis 135°C/0,05 mbar; |
| NCO-Wert: | 28,3%; |
| Viskosität: | 110 mPa · s/25°C; |
| Gehalt an hydrolysierbarem Chlor: | 0,02%. |

### Beispiel 2

a) Analog Beipiel 1a) werden 250 g (1,11 Mol) 4,4'-Diamino-3,5-dimethyldiphenylmethan in Gegenwart von 25 g Ruthenium-Aluminumoxid-Trägerkatalysator und 25 g Ammoniak bei 140°C und 200 bar hydriert bis 3,3 Mol Wasserstoff abreagiert sind. Nach Reinigung durch Flash-Destillation und Fein-Destillation bei 150 bis 155°C/0,1 bis 0,2 mbar werden 200 g Produkt gewonnen. Es

enthält gemäß gaschromatographischer Analyse 97,4% 4 (4-Amino-3,5-dimethylbenzyl)-cycloh exylamin, 2,1% 4,4'-Diamino-3,5-dimethyldicyclohexylmethan und 0,5% nicht-umgesetztes Ausgangsprodukt.

b) In eine Lösung von 200 g Phosgen in 700 ml wasserfreiem Chlorbenzol läßt man bei 0 bis 8° C 116 g des gemäß Beispiel 2a) hergestellten Diamingemisches gelöst in 700 ml Chlorbenzol unter intensivem Rühren eintropfen. Man erwärmt die entstandene Suspension unter Einleiten von 100 g/h Phosgen auf 120° C. Dabei löst sich der Feststoff langsam auf und bei 65° C entsteht eine klare Lösung. Nach zweistündigem Phosgenieren bei 120° C wird entphosgeniert und das Rohprodukt durch Destillation gereinigt. Dabei werden 121 g (87,5% der Theorie) 4-(4-Isocyanato-3,5-dimethylbenzyl)-cyclohexylisocyanat mit einem Siedepunkt von 138 bis 141° C/0,1 mbar, einem NCO-Gehalt von 29,5% und einer Viskosität von 140 mPa · s/25° C gewonnen.

Beispiel 3

a) Man beschickt einen 0,7 l Rührautoklaven mit 250 g (0,98 Mol) 4,4'-Diamino-3,5-diethyldiphenylmethan, 25 g Ruthenium-Aluminiumoxid-Trägerkatalysator (5% Ru auf $Al_2O_3$), spült mit Stickstoff und dosiert 25 g Ammoniak zu. Danach hydriert man bei 140° C und 200 bar bis 3 Mol Wasserstoff umgesetzt sind. Man läßt den Autoklaven abkühlen, entspannt, nimmt das Produkt in Methanol auf und filtriert den Katalysator ab. Anschließend wird das Lösungsmittel abgezogen und das hydrierte Diamin durch Flash- und Fein-Destillation bei 136 bis 140° C/0,1 mbar gereinigt. Man erhält 170 g Diamin mit einem Gehalt von 95,8% 4-(4-Amino-3,5-diethylbenzyl)-cyclohexylamin, 3,3% perhydriertem Ausgangsdiamin und 0,9% nicht-umgesetztem Ausgangsprodukt. Die Analyse erfolgt durch Gaschromatographie.

b) Man löst 200 g Phosgen in 700 ml wasserfreiem Chlorbenzol und läßt eine Lösung von 130 g Diamin aus Beispiel 3a) in 700 ml trockenem Chlorbenzol unter Rühren und Kühlen in die Phosgenlösung eintropfen. Dabei entsteht eine schwer rührbare Dispersion, die beim Erwärmen unter Einleiten von Phosgen bei 80° C in eine klare Lösung übergeht. Man kocht 2 Stunden am Rückfluß, beendet die Phosgenzufuhr, entphosgeniert und destilliert das Rohprodukt bei 135° C/0,05 mbar. Man erhält 134 g (90% der Theorie) 4-(4-Isocyanato-3,5-diethylbenzyl)-cyclohexylisocyanat, mit einem NCO-Gehalt von 27,0% und einer Viskosität von 90 mPa · s/25° C.

Beispiel 4

a) 250 g (0,89 Mol) 4,4'-Diamino-3,5-diisopropyldiphenylmethan werden analog Beispiel 1a) in Gegenwart von 25 g Ruthenium-Aluminiumoxid-Trägerkatalysator (5% Ru auf $Al_2O_3$) und 25 g Ammoniak bei 140° C/200 bar hydriert bis 2,7 Mol Wasserstoff absorbiert sind. Nach Flash-Destillation wird das Produkt einer fraktionierten Destillation unterworfen. Dabei fallen in der Hauptfraktion 214 g 4-(4-Amino-3,5-diisopropylbenzyl)-cyclohexylamin an, das gemäß gaschromatographischem Befund 0,5% an perhydriertem Diamin und 0,5% Ausgangsdiamin enthält.

b) Eine Lösung von 200 g Phosgen in 700 ml trockenem Chlorbenzol wird analog Beispiel 1a) mit einer Lösung von 144 g (0,5 Mol) 4-(4-Amino-3,5-diisopropylbenzyl-cyclohexylamin versetzt. Unter Phosgenzufuhr wird das Gemisch auf Rückflußtemperatur gebracht und 2 Stunden gekocht. Man entphosgeniert und reinigt das Produkt durch Destillation unter vermindertem Druck. Die Ausbeute an 4-(4-Isocyanato-3,5-diisopropylbenzyl)-cyclohexylisocyanat beträgt 154 g. Das Produkt ist durch folgende Daten charakterisiert:

| | |
|---|---|
| NCO-Gehalt: | 24,6% |
| Siedepunkt: | 170—190° C/0,1 mbar |
| hydrolysierbares Chlor: | 0,05% |
| Viskosität: | 500 mPa · s/25° C |

Beispiel 5

a) Man hydriert gemäß Beispiel 1a) 250 g (1,18 Mol) 4,4'-Diamino-3-methyldiphenylmethan in Gegenwart von 25 g eines handelsüblichen Ruthenium-Katalysators (5 Gew.-% Ruthenium auf $Al_2O_3$) und 25 g Ammoniak. Das Rohprodukt wird in Methanol aufgenommen, der Katalysator abfiltriert, gewaschen und die vereinigten Lösungen unter vermindertem Druck fraktioniert destilliert. Dabei werden in der Hauptfraktion 210 g Diamin mit einem Siedepunkt von 125 bis 135° C/0,1 mbar gewonnen, das neben 85,0% 4-(4-Amino-3-methylbenzyl)-cyclohexyamin, 13% 4,4'-Diaminodicyclohexylmethan und 2% 4,4'-Diaminodiphenylmethan enthält.

b) Eine Lösung von 400 g Phosgen in 1300 ml trockenem Chlorbenzol wird bei —5 bis 8° C vorgelegt. Unter Kühlen und Rühren läßt man 210 g des Diamins aus Beispiel 5a) gelöst in 1300 ml Chlorbenzol zutropfen und heizt die entstandene Suspension unter Einleiten von 100 g/h Phosgen auf 130° C. Bei 50 bis 60° C schmilzt der Feststoff, es bildet sich eine Emulsion, die bei 75° C in eine klare Lösung übergeht. Man phosgeniert 1,5 Stunden bei 130° C, entphosgeniert und reinigt das entstandene 4-(4-Isocyanato-3-methylbenzyl)-cyclohexylisocyanat durch Destillation. Dabei wurden 188 g (85% der Theorie) bei 130 bis 133° C/0,5 mbar siedendes Diisocyanat gewonnen, das laut gaschromatographischem Befund eine Reinheit von 98%, einen NCO-Gehalt von 30,8% und eine Viskosität von 50 mPa · s/25° C aufweist.

Beispiel 6

a) In einem 0,7 l Rührautoklaven werden 266 g (1 Mol) 4,2'-Diamino-3,5-dimethyldiphenylmethan und 22,6 g Ruthenium-Aluminiumoxid-Trägerkatalysator (5%Ru) vorgelegt. Nach mehrmaligem Spülen mit Stickstoff und Wasserstoff werden 22,6 g Ammoniak zudosiert, das Gemisch auf 140° C erwärmt und unter Rühren bei 200 bar Wasserstoff hydriert bis nach vierstündiger Reaktionszeit 3 Mol Wasserstoff absorbiert sind. Man stoppt die Reaktion durch Abstellen des Rührers, läßt auf Raumtemperatur abkühlen, entspannt den Autoklaven und nimmt das Produkt in Methanol auf. Der Katalysator wird nun abfiltriert, mit Methanol gewaschen und die vereinigten Lösungen einer Flashdestillation unterworfen. Bei einer Siedetemperatur von 114 bis 152° C0,015 mbar gehen 228,6 g Diamin über, das zu 93,0% aus 2-(4-Amino-3,5-dimethylbenzyl)-cyclohexylamin, 6,3% 4,2'-Diamino-3,5-dimethyldicyclohexylmethan und 0,6% aus nicht-umgesetztem Ausgangsprodukt besteht.

b) Man löst 200 g Phosgen in 430 ml wasserfreiem Chlorbenzol bei 0 bis 8° C und läßt unter Rühren und Kühlen eine Lösung von 116 g des Diamingemisches aus Beispiel 6a) in 500 ml wasserfreiem Chlorbenzol so zutropfen, daß die Temperatur des Reaktionsgemisches 10° C nicht übersteigt. Anschließend erwärmt man unter Einleiten von 120 g/h Phosgen auf 120° C, wobei sich die entstandene Suspension löst, und rührt noch 3 Stunden unter gleichen Bedingungen nach. Nach zweistündigem Entphosgenieren wird das Produkt durch Flashdestillation und Feindestillation bei 126° C/0,06 mbar gereinigt.

Die Ausbeute beträgt 127,4 g (96% der Theorie) 2-(4-Isocyanato-3,5-dimethylbenzyl)-cyclohexylisocyanat, das einen NCO-Gehalt von 29,6%, einen Gehalt an hydrolisierbarem Chlor von 0,01% und eine Viskosität von 110 mPa · s/25° C aufweist.

Beispiel 7

a) Man beschickt einen 0,7 l Rührautoklaven mit 212 g (1 Mol) 4,2'-Diamino-3-methyldiphenylmethan und 21,2 g Ruthenium-Aluminiumoxid-Trägerkatalysator (Ru-Gehalt 5%) und dosiert nach Spülen mit Stickstoff und Wasserstoff 21,2 g Ammoniak zu. Nun hydriert man unter Rühren bei einer Temperatur von 140° C und einem Druck von 200 mbar bis 3 Mol Wasserstoff absorbiert sind. Dann läßt man den Autoklaven auf Raumtemperatur abkühlen, entspannt und nimmt das Rohprodukt in Methanol auf. Der Katalysator wird durch Filtration abgetrennt, mit Methanol gewaschen und das Produkt destillativ gereinigt. Bei einer Temperatur von 145 bis 149° C/0,018 mbar destillieren 153,4 g 2-(4-Amino-3-methylbenzyl)-cyclohexylamin, das laut gaschromatographischem Befund eine Reinheit von 98,4% aufweist.

b) 109 g 2-(4-Amino-3-methylbenzyl)-cyclohexylamin aus Beispiel 7a), gelöst in 500 ml wasserfreiem Chlorbenzol, läßt man unter Rühren und Kühlen in eine Lösung von 200 g Phosgen in 430 ml wasserfreiem Chlorbenzol tropfen. Danach erwärmt man die entstandene Suspension unter Einleiten von Phosgen auf Rückflußtemperatur und kocht weitere 4 Stunden. Man entphosgeniert 2 Stunden, destilliert das Lösungsmittel unter vermindertem Druck ab und reinigt das Rohprodukt durch zweimalige Destillation. Man erhält 122,6 g (92% der Theorie) 2-(4-Isocyanato-3-methylbenzyl)-cyclohexylisocyanat, das durch folgende Daten charakterisiert ist:

| | |
|---|---|
| Siedepunkt: | 135° C/0,05 mbar |
| NCO-Wert: | 31,0% |
| Gehalt an hydrolisierbarem Chlor: | 0,03% |
| Viskosität: | 50 mPa · s/25° C |

**Patentansprüche**

1. Gegebenenfalls Isomerengemische darstellende und gebenenfalls im Gemisch mit untergeordneten Mengen an den entsprechenden perhydrierten Diisocyanaten und/oder im Gemisch mit untergeordneten Mengen an den entsprechenden unhydrierten aromatischen Diisocyanaten vorliegende Diisocyanate der Formel

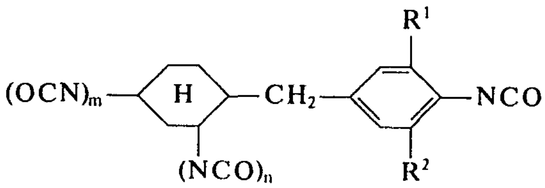

in welcher

$R^1$ und $R^2$ jeweils gleich oder verschieden sind und Wasserstoff oder (gegebenenfalls verzweigte) Alkylgruppen mit 1 bis 12 Kohlenstoffatomen bedeuten mit der Maßgebe, daß mindestens einer der Reste $R^1$ und $R^2$ für einen Alkylrest steht und

m und n jeweils für 0 oder 1 stehen, mit der Maßgabe, daß die Summe $m + n$ den Wert 1 ergibt, wobei im Falle von m oder $n = 0$ die verbleibende freie Valenz durch Wasserstoff abgesättigt ist.

2. Diisocyanate gemäß Anspruch 1 der allgemeinen Formel

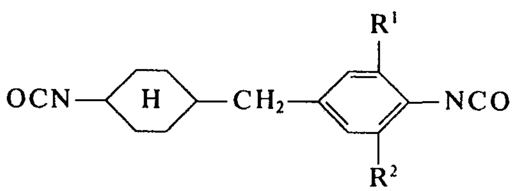

dadurch gekennzeichnet, daß

$R^1$ und $R^2$ für gleiche oder verschiedene Reste stehen und jeweils (gegebenenfalls verzweigte) Alkylgruppen mit 1 bis 4 Kohlenstoffatomen bedeuten.

3.Diisocyanate gemäß Anspruch 1 der allgemeinen Formel

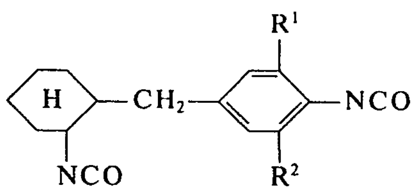

dadurch gekennzeichnet, daß

$R^1$ und $R^2$ für gleiche oder verschiedene Reste stehen und jeweils (gegebenenfalls verzweigte) Alkylgruppen mit 1 bis 4 Kohlenstoffatomen bedeuten.

4. Diisocyanat gemäß Anspruch 1 der Formel

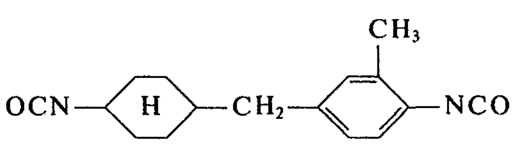

5. Diisocyanat gemäß Anspruch 1 der Formel

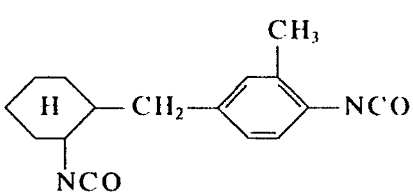

6. Verfahren zur Herstellung von Diisocyanaten oder Diisocyanatgemischen gemäß Anspruch 1 bis 5, dadurch gekennzeichnet, daß man die ihnen zugrundeliegenden, gegebenenfalls als Stellungs- und/ oder Stereoisomerengemisch und gegebenenfalls im Gemisch mit untergeordneten Mengen an den entsprechenden perhydrierten Diaminen und/oder gegebenenfalls im Gemisch mit untergeordneten Mengen an den entsprechenden unhydrierten aromatischen Diaminen vorliegenden Aminobenzyl-cyclohexylamine in an sich bekannter Weise bei $-20$ bis $+250^{\circ}$C phosgeniert.

7. Verwendung der Diisocyanate bzw. Diisocyanatgemische gemäß Anspruch 1 bis 5 als Ausgangsmaterialien bei der Herstellung von Polyurethankunststoffen nach dem Isocyanat-Polyadditionsverfahren.

**Claims**

1. Diisocyanates which are optionally isomer mixtures and are optionally present in admixture with minor quantities of the corresponding perhydrogenated diisoyanates and/or in admixture with minor quantities of the corresponding unhydrogenated aromatic diisocyanates and which have the formula

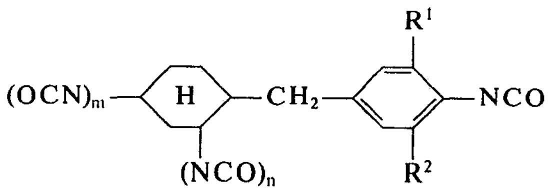

in which

$R^1$ and $R^2$ are identical or different in each case and denote hydrogen or (optionally branched) alkyl groups with 1 to 12 carbon atoms, with the proviso that at least one of the radicals $R^1$ and $R^2$ represents an alkyl radical and

m and n in each case represent 0 or 1, with the proviso that the sum of $m+n$ equals 1, and in the case where m or $n=0$ the remaining free valency is saturated by hydrogen.

2. Diisocyanates according to Claim 1 of the general formula

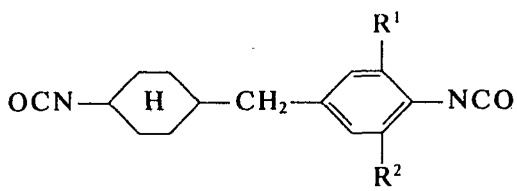

characterised in that

$R^1$ and $R^2$ represent identical or different radicals and in each case denote (optionally branched) alkyl groups with 1 to 4 carbon atoms.

3. Diisocyanates according to Claim 1 of the general formula

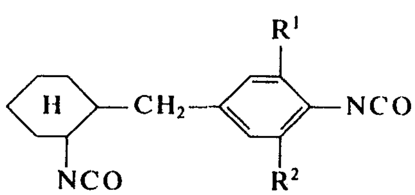

characterised in that

$R^1$ and $R^2$ represent identical or different radicals and in each case denote (optionally branched) alkyl groups with 1 to 4 carbon atoms.

4. Diisocyanate according to Claim 1 of the formula

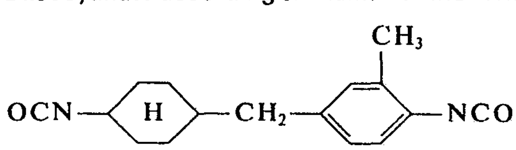

5. Diisocyanate according to Claim 1 of the formula

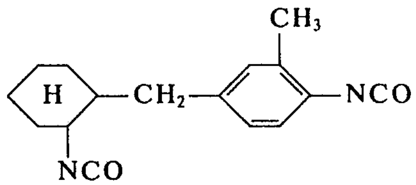

6. Process for the production of diisocyanates or diisocyanate mixtures according to Claim 1 to 5, characterised in that the aminobenzylcyclohexylamines on which they are based and which are optionally present as position and/or stereoisomer mixtures and are optionally in admixture with minor quantities of the corresponding perhydrogenated diamines and/or optionally in admixture with minor quantities of the corresponding unhydrogenated aromatic diamines are phosgenated in a manner known per se at −20 to +250° C.

7. Use of the diisocyanates or diisocyanate mixtures according to Claim 1 to 5 as starting materials in the production of polyurethane plastics according to the isocyante-polyaddition process.

**Revendications**

1. Diisocyanates formant éventuellement des mélanges d'isomères et présents le cas échéant en mélange avec de faibles quantités des diisocyanates perhydrogénés correspondants et/ou en mélange avec de faibles quantités des diisocyanates aromatiques von hydrogénés correspondants, de formule

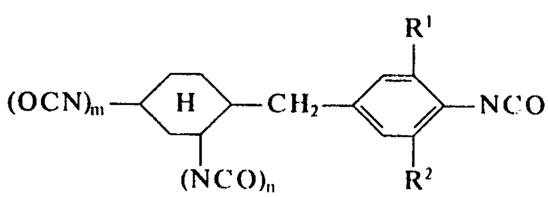

dans laquelle

$R^1$ et $R^2$ sont, dans chaque cas, égaux ou différents et désignent l'hydrogène ou des groupes alkyle (éventuellement ramifiés) ayant 1 à 12 atomes de carbon, à condition qu'au moins l'un des restes $R^1$ et $R^2$ soit un reste alkyle, et

m et n sont égaux chacun à 0 or à 1, à condition que la somme m + n donne la valeur 1, et au cas où m ou n est égal à 0, la valence libre restante est saturée par de l'hydrogène.

2. Diisocyanates suivant la revendication 1, de formule générale

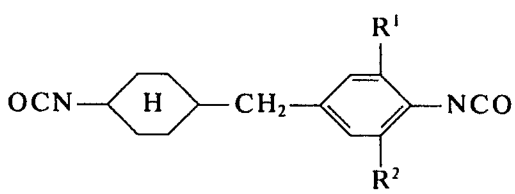

caractérisés en ce que

$R^1$ et $R^2$ sont des restes égaux ou différents et désignent chacun des groupes alkyle (éventuellement ramifiés) ayant 1 à 4 atomes de carbone.

3. Diisocyanates suivant la revendication 1, de formule générale

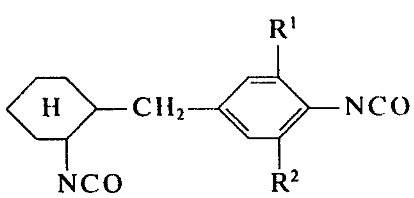

caractérisés en ce que

$R^1$ et $R^2$ sont des restes égaux ou différents et désignent chacun des groupes alkyle (éventuellement ramifiés) ayant 1 à 4 atomes de carbone.

4. Diisocyanate suivant la revendication 1, de formule

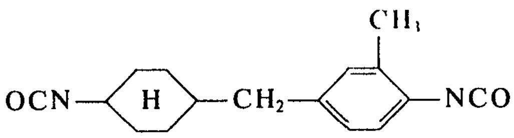

5. Diisocyanate suivant la revendication 1, de formule

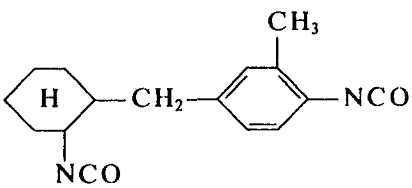

6. Procédé de production de diisocyanates ou de mélanges de diisocyanates suivant les revendications 1 à 5, caractérisé en ce qu'on phosgène d'une manière connue à une température de −20 à +250°C les aminobenzylcyclohexylamines qui leur servent de base et qui se présentent éventuellement sous la forme d'un mélange d'isomères de position et/ou de stéréo-isomères et éventuellement en mélange avec de faibles quantités des diamines perhydrogénées correspondantes et/ou, le cas échéant, en mélange avec de faibles quantités des diamines aromatiques non hydrogénées correspondantes.

7. Utilisation des diisocyanates et mélanges de diisocyanates suivant les revendications 1 à 5 comme matières de départ dans la production de matières plastiques du type polyuréthanne par le procédé de polyaddition d'isocyanate.